Europäisches Patentamt

European Patent Office

Office européen des brevets

(11) Veröffentlichungsnummer: **0 480 438 A1**

# (12) EUROPÄISCHE PATENTANMELDUNG

(21) Anmeldenummer: **91117321.9**

(51) Int. Cl.5: **C07C 335/28**

(22) Anmeldetag: **10.10.91**

(30) Priorität: **11.10.90 DE 4032324**

(43) Veröffentlichungstag der Anmeldung:
**15.04.92 Patentblatt 92/16**

(84) Benannte Vertragsstaaten:
**AT BE DE FR GB IT NL**

(71) Anmelder: **SKW TROSTBERG
AKTIENGESELLSCHAFT
Dr.-Albert-Frank-Strasse 32
W-8223 Trostberg(DE)**

(72) Erfinder: **Hintermaier, Helmut, Dr.
Mühlbachweg 12
W-8223 Trostberg(DE)**
Erfinder: **Maier, Ursula
Kraftwerkstrasse 3
W-8221 Tacherting(DE)**

(74) Vertreter: **Huber, Bernhard, Dipl.-Chem. et al
Patentanwälte H. Weickmann, Dr. K. Fincke
F.A. Weickmann, B. Huber Dr. H. Liska, Dr. J.
Prechtel Kopernikusstrasse 9 Postfach 86 08
20
W-8000 München 86(DE)**

(54) **Verfahren zur Herstellung von Guanylthioharnstoff.**

(57) Die vorliegende Erfindung betrifft ein Verfahren zur Herstellung von Guanylthioharnstoff durch Umsetzung von Dicyandiamid mit Schwefelwasserstoff und N-Alkylpyrrolidon als Lösemittel in Anwesenheit katalytisch wirkender Zusätze, von Aminen, Ammoniak und/oder elementarem Schwefel, Zersetzung der gebildeten Additionsverbindungen von Guanylthioharnstoff und N-Alkylpyrrolidon durch Behandlung mit einem organischen Lösemittel wie z. B. Toluol und Gewinnung des Guanylthioharnstoffs als Rückstand.

EP 0 480 438 A1

Die Erfindung betrifft ein Verfahren zur Herstellung von Guanylthioharnstoff durch Umsetzung von Dicyandiamid mit Schwefelwasserstoff in einem organischen Lösemittel und dessen Isolierung.

Es ist bekannt, daß Dicyandiamid in wäßrigem oder alkoholischem Medium Schwefelwasserstoff anlagert unter Bildung von Guanylthioharnstoff. Trotz langer Reaktionszeiten oder Durchführung der Reaktion unter Druck sind Ausbeute und Reinheit, insbesondere infolge der Bildung von Dithiobiuret, unbefriedigend.

Die DE-OS 16 70 146 beschreibt ein Verfahren zur Herstellung von Additionsverbindungen von N-Alkylpyrrolidonen mit Guanylthioharnstoff. Während die Ausbeute an Additionsprodukt mit 93 % befriedigend ist, geht beim beschriebenen Umkristallisieren mit Alkohol viel des gewünschten Produktes verloren, so daß dieses Verfahren für eine Anwendung im technischen Maßstab ungeeignet erscheint.

Der Erfindung liegt daher die Aufgabe zugrunde, ein Verfahren zur Herstellung von Guanylthioharnstoff zur Verfügung zu stellen, welches die beschriebenen Nachteile vermeidet und es erlaubt, Guanylthioharnstoff in guter Ausbeute und hoher Reinheit herzustellen.

Gelöst wird diese Aufgabe erfindungsgemäß durch Umsetzung von Dicyandiamid mit Schwefelwasserstoff in N-Alkylpyrrolidon als Lösemittel in Anwesenheit katalytisch wirkender Zusätze, dadurch gekennzeichnet, daß man als katalytisch wirkenden Zusatz ein Amin, Ammoniak oder/und elementaren Schwefel verwendet, unter Bildung einer Additionsverbindung von Guanylthioharnstoff und N-Alkylpyrrolidon, diese durch Behandlung mit einem organischen Lösemittel zersetzt und den Guanylthioharnstoff als Rückstand gewinnt.

Als Dicyandiamid kann man das technische Produkt verwenden, das in Reinheiten über 98,5 % auf dem Markt erhältlich ist. Der Schwefelwasserstoff kann in reiner Form, gemischt mit Inertgas oder als ein technisch anfallendes Gemisch von Schwefelwasserstoff und Kohlendioxid verwendet werden. Das Verhältnis von Schwefelwasserstof zu Kohlendioxid kann hierbei im Bereich von 70 : 30 bis 30 : 70 schwanken.

Als Reaktionsmedium für die Umsetzung des Dicyandiamids mit dem Schwefelwasserstoff dient ein N-Alkylpyrrolidon. N-Methylpyrrolidon wird bevorzugt, andere N-Alkylpyrrolidone wie N-Ethyl- oder monomeres N-Vinylpyrrolidon sind aber ebenfalls mit gutem Erfolg einsetzbar. Das N-Alkylpyrrolidon kann als alleiniges Reaktionsmedium verwendet werden oder im Gemisch mit anderen Lösungsmitteln. Besonders hat sich der Zusatz von Toluol oder Xylol bewährt, wobei zweckmäßig ein Gewichtsverhältnis von N-Alkylpyrrolidon zu Toluol oder/und Xylol von 1 : 1 bis 1 : 1,5 eingesetzt wird. Erfindungswesentlich ist es, dem Reaktionsmedium die erwähnten, die Reaktion zwischen Dicyandiamid und Schwefelwasserstoff begünstigenden Zusätze zuzufügen. Hierfür haben sich primäre, sekundäre und tertiäre Amine wie z.B. Methyl-, Diethyl- oder Tributylamin bewährt. Besonders bewährt hat sich die Zugabe von Ammoniak, vorzugsweise in einer Menge von 0,2 bis 1,5 Gew.-%, besonders bevorzugt von 0,2 bis 0,6 Gew.-% bezogen auf das eingesetzte Dicyandiamid. Es hat sich außerdem überraschenderweise herausgestellt, daß neben Ammoniak als Katalysator auch noch zusätzlich elementarer Schwefel, bevorzugt in einer Menge von 0,5 bis 3,0 Gew.-% bezogen auf eingesetztes Dicyandiamid, die Reaktionsgeschwindigkeit ganz wesentlich beeinflußt und die Bildung unerwünschter Nebenprodukte weitgehend zurückdrängt.

Schließlich wurde gefunden, daß einen besonders starken katalytischen Einfluß auf die Reaktion zurückgeführte, ungereinigte Mutterlauge ausübt, wie in der Versuchsreihe Nr. 3 gezeigt wird. Es werden über 10 Ansätze hinweg praktisch quantitative Ausbeuten erhalten, wobei die Reinheit des Guanylthioharnstoffs in allen Fällen über 98 % lag. Damit ist das Verfahren hervorragend geeignet, kontinuierlich betrieben zu werden, da kaum Nebenprodukte gebildet werden, die ausgeschleust werden müssen.

Die Reaktionstemperatur liegt zweckmäßig zwischen 70 und 120° C; bevorzugt werden etwa 90° C eingehalten, wobei günstige Reaktionsgeschwindigkeiten bei entsprechenden Umsetzungsgraden erhalten werden. Die Reaktionsdauer liegt unter optimalen Bedingungen zwischen 3 und 4,5 Stunden. Längere Reaktionszeiten bis zu 10 Stunden können notwendig werden, wenn z. B. Verschiebungen in dem die Reaktion begünstigenden Katalysatorsystem auftreten.

Das zunächst als Zwischenprodukt anfallende Additionsprodukt von N-Alkylpyrrolidon und Guanylthioharnstoff kann aus dem Reaktionsmedium durch Abkühlen in kristalliner Form gewonnen werden. Es kann dann mit einer kleinen Menge der gleichen Lösemittelkombination, die für die Herstellung verwendet wurde, nachgewaschen werden. Anschließend wird der Komplex mit einem wasserfreien heißen Extraktionsmittel behandelt, wobei überraschenderweise nur das N-Alkylpyrrolidon in Lösung geht und der Guanylthioharnstoff als praktisch farbloses kristallines Produkt zurückbleibt.

Als Extraktionsmittel hat sich Toluol bewährt. Es können auch Gemische von Toluol mit einem oder mehreren anderen Arenderivaten wie z. B. Xylol, Anisol, Chlorbenzol oder Toluol oder/und Estern wie z. B. Kohlensäuredialkylestern, Ketonen wie z. B. Diethylketon oder Methylisobutylketon, eingesetzt werden.

Das Mischungsverhältnis von Toluol zur anderen Lösemittelkomponente kann in weiten Grenzen variieren und kann 100 : 0 bis 50 : 50 betragen.

2

Guanylthioharnstoff dient als Zwischenprodukt zur Herstellung von Triazinen, optischen Aufhellern und Pflanzenschutzmitteln sowie als Komponente in Düngemitteln.

Die nachfolgenden Beispiele sollen die Erfindung näher erläutern.

Beispiel 1

In einem mit Rührer, Thermometer, Kühler und Gaseinleitungsrohr ausgestatteten Rührgefäß werden zu 130 Gew.-Teilen Toluol und 99 Gew.-Teilen N-Methylpyrrolidon 84 Gew.-Teile Dicyandiamid, 0,1 Gew.-Teile Tributylamin und 1 Gew.-Teil Guanylthioharnstoff zugegeben. Nach Spülen des Reaktionsgefäßes mit Stickstoff wird aufgeheizt und bei etwa 70°C mit dem Einleiten von Schwefelwasserstoff begonnen. Die Temperatur wurde auf 90°C gesteigert und das Einleiten von Schwefelwassersstoff so lange fortgesetzt, bis die Aufnahme der theoretischen Menge von 34 Gew.-Teilen nach etwa 10 Stunden beendet war.

Die gebildete Additionsverbindung von Guanylthioharnstoff/N-Methylpyrrolidon wurde unter Rühren abgekühlt, abgesaugt und mit etwas N-Methylpyrrolidon/Toluol nachgewaschen.

Durch Extraktion mit heißem Toluol, zuerst bei 60°C, dann bei 85°C wurde die Additionsverbindung getrennt. Durch kurzzeitiges Rückflußkochen des Rückstandes und nochmaliges Abfiltrieren wurde der Guanylthioharnstoff als praktich farbloses Produkt in einer Reinheit von über 98 %, mit einem Schmelzpunkt von 155 bis 164°C, erhalten. Die Ausbeute betrug 114 g, entspr. 96,5 %.

Beispiel 2

Entsprechend Beispiel 1 wurden folgende Ansätze mit anderen Katalysatoren und anderem Lösemittelgemisch durchgeführt.

| Ver-suchs-Nr. | Kataly-sator (Gew.-Tle.) | Dicyan-diamid (Gew.-Tle.) | Lösungs-mittel (Gew.-Tle.) | Reak-tions-zeit (Std.)/ Tempera-tur (°C) | Aus-waage (Gew.-Tle.) | Aus-beute- (%) |
|---|---|---|---|---|---|---|
| 1 | 0,8 GTH | 84 | 247,5 NMP | 8/90 | 107,2 | 90,7 |
| 2 | 0,1 TBA 0,8 GTH | 84 | 99 NMP 150 Toluol | 9/88 | 112,8 | 95,5 |
| 3 | 0,03 TBA 0,8 GTH | 84 | 25⎤ 25⎦ NMP 150 Toluol | 12/90 | 112,5 | 95,2 |
| 4 | 0,06 TBA 0,8 GTH | 84 | 50 NMP 150 Toluol | 16/85 | 112,8 | 95,5 |
| 5 | 0,50 NH$_3$ 2 S | 84 | 150 NMP 100 Toluol | 4,8/86 | 114,3 | 96,7 |
| 6 | 3 S | 84 | 99 NMP 130 Toluol | 4,5/90 | 114,7 | 97,1 |

GTH = Guanylthioharnstoff

TBA = Tributylamin

NMP = N-Methylpyrrolidon

S   = Schwefel

Die Beispiele zeigen die deutliche Reaktionsbeschleunigung durch Anwendung von Ammoniak und/oder Schwefel als Katalysatorsystem bei der Herstellung von Guanylthioharnstoff.

Die Additionsverbindung wurde jeweils durch Extraktion mit Toluol getrennt. Analoge Ergebnisse wurden mit Gemischen von Toluol mit verschiedenen Butanolderivaten, Ketonen oder Estern wie z. B. Kohlensäuredialkylestern im Verhältnis Toluol zu anderer Lösungsmittelkomponente wie 1 : 1 erhalten.

Beispiel 3

In dieser Reihe von 10 Versuchen wird der Einfluß der rückgeführten Mutterlauge auf die Bildung von Guanylthioharnstoff beschrieben.

4

Es wurde ein Reihenversuch mit 8 Ansätzen unter Wiedereinsatz der Mutterlauge bei 90°C durchgeführt. Die Ausgangsreaktionsmischung (Versuch 1) enthielt 84 Gew.-Teile Dicyandiamid, 150 Gew.-Teile Toluol, 99 Gew.-Teile N-Methylpyrrolidon (NMP) und 0,1 % Tributylamin und 0,8 % Guanylthioharnstoff bezüglich Dicyandiamid.

Für die Versuche 2 bis 8 wurde die in der folgenden Tabelle angegebene Menge an Toluol (jeweils 150 Gew.-Teile) zusammen mit der jeweils angegebenen Menge an Mutterlauge des vorherigen Versuchs (= Rückstand des Reaktionsmediums nach Abdestillation von Toluol und Abtrennung des Produkts) zu frischem Dicyandiamid gegeben, wobei zusätzlich in jedem Versuch 3,5 g frisches NMP zugesetzt wurden.

| Ver-suchs-Nr. | Kata-lysa-tor (Gew.-Tle.) | Reak-tions-medium (Gew.-Tle.) | Mut-ter-lauge (Gew.-Tle.) | $H_2S$-Auf-nahme (Gew.-Tle.) | Reak-tions-zeit (Std.) | Aus-waage (Gew.-Tle.) | Aus-beute (%) |
|---|---|---|---|---|---|---|---|
| 1 | – | 99 NMP 150 Toluol | – | 30 | 11 | 108,9 | 92,2 |
| 2 | – | 150 | 93,3 | 35,5 | 4,3 | 116,5 | 98,6 |
| 3 | – | 150 | 94 | 33,4 | 3,5 | 117,7 | 99,6 |
| 4 | – | 150 | 100 | 34,5 | 3,5 | 116,8 | 98,8 |
| 5 | – | 150 | 102 | 32,4 | 4,4 | 118,2 | 100,0 |
| 6 | – | 150 | 109 | 33,5 | 3,8 | 117 | 99,0 |
| 7 | – | 150 | 112 | 32,2 | 3,7 | 117,7 | 99,6 |
| 8 | – | 150 | 112 | 34,7 | 4,6 | 117,9 | 99,8 |

NMP = N-Methylpyrrolidon

Bei den Versuchen 6 bis 8 wurde Schwefelwasserstoff im Gemisch mit 40 Vol.-Teilen Kohlendioxid verwendet.

Beispiel 4

Entsprechend Beispiel 3 wurde ein Reihenversuch mit $NH_3$ als Katalysator unter Wiedereinsatz der Mutterlauge durchgeführt. Die Ausgangsreaktionsmischung war wie in Beispiel 3, außer daß 1,2 Gew.-Teile $NH_3$ als Katalysator verwendet wurden. In Ansatz 2 wurden der Mutterlauge zusätzlich 2,0 Gew.-Teile $NH_3$ und 0,5 Gew.-Teile frisches NMP zugesetzt. In Ansatz 3 wurden zusätzlich 2,2 Gew.-Teile $NH_3$ und 0,5 Gew.-Teile frisches NMP eingesetzt.

Die Reaktionstemperatur war 90°C. Die Einleitung von $H_2S$ erfolgte in Form eines Gemisches mit 50 Vol.-% $CO_2$.

Tabelle

| Ver- suchs- -Nr. | Kata- lysa- tor (Gew. -Tle.) | Reak- tions- medium (Gew. -Tle.) | Mut- ter- lauge (Gew. -Tle.) | $H_2S$- -Auf- nahme (Gew. -Tle.) | Reak- tions- zeit (Std.) | Aus- waage (Gew. -Tle.) | Aus- beute (%) |
|---|---|---|---|---|---|---|---|
| 1 | 1,2 | 99 NMP 150 Toluol | – | $31,5[1]$ | 12,2 | 110,9 | 93,8 |
| 2 | 2,0 | 150 | 89,8 | 33,6 | 7,5 | 118,1 | 99,9 |
| 3 | 2,2 | 150 | 96,1 | n.b. | 5,3 | 118,0 | 99,9 |

[1] Mindermengen resultieren aus der Flüchtigkeit von Toluol

**Patentansprüche**

1. Verfahren zur Herstellung von Guanylthioharnstoff durch Umsetzung von Dicyandiamid mit Schwefelwasserstoff in N-Alkylpyrrolidon als Lösemittel in Anwesenheit katalytisch wirkender Zusätze,
   **dadurch gekennzeichnet,**
   daß man als katalytisch wirkenden Zusatz ein Amin, Ammoniak oder/und elementaren Schwefel verwendet, unter Bildung einer Additionsverbindung von Guanylthioharnstoff und N-Alkylpyrrolidon, diese durch Behandlung mit einem organischen Lösemittel zersetzt und den Guanylthioharnstoff als Rückstand gewinnt.

2. Verfahren nach Anspruch 1,
   **dadurch gekennzeichnet,**
   daß man als Reaktionsmedium ein Gemisch von N-Methylpyrrolidon und Toluol mit einem Gehalt an Ammoniak und elementarem Schwefel verwendet.

3. Verfahren nach Anspruch 2,
   **dadurch gekennzeichnet,**
   daß die Gehalte von Ammoniak 0,2 bis 1,5 Gew.-%, die des Schwefels 0,5 bis 3,0 Gew.-% bezogen auf eingesetztes Dicyandiamid betragen.

4. Verfahren nach den Ansprüchen 1 bis 3,
   **dadurch gekennzeichnet,**
   daß man ein Gemisch von Schwefelwasserstoff mit Kohlendioxid im Volumenverhältnis von 70 : 30 bis 30 : 70 verwendet.

5. Verfahren nach den Ansprüchen 1 bis 4,
   **dadurch gekennzeichnet,**
   daß man die Mutterlauge rezyclisiert.

**6.** Verfahren nach den Ansprüchen 1 bis 5,
**dadurch gekennzeichnet,**
daß man das Additionsprodukt von Guanylthioharnstoff und N-Methylpyrrolidon mit Toluol zersetzt.

**7.** Verfahren nach Anspruch 6,
**dadurch gekennzeichnet,**
daß man Toluol im Gemisch mit wenigstens einem weiteren Aren-, Ester- oder Ketonderivat im Mischungsverhältnis 100 : 0 bis 50 : 50 verwendet.

# EUROPÄISCHER RECHERCHENBERICHT

Nummer der Anmeldung

**EP 91 11 7321**

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| A,D | DE-A-1 670 146  (BASF A.G.) <br> * das ganze Dokument * * <br> – – – | 1-7 | C 07 C 335/28 |
| A | CHEMICAL ABSTRACTS, vol. 75, no. 15, 11. Oktober 1971, Columbus, Ohio, US; abstract no. 98198S, Seite 285 ; <br> * Zusammenfassung * <br> & JP-A-46 019 939 (NITTO CHEMICAL INDUSTRIAL CO LTD.) * <br> – – – | 1-7 | |
| A | CHEMICAL ABSTRACTS, vol. 94, no. 23, 8. Juni 1981, Columbus, Ohio, US; abstract no. 191733P, Seite 603 ; <br> * Zusammenfass <br> & JP-A-56 002 958 (NIPPON CARBIDE INDUSTRIES CO. ) * <br> – – – | 1-7 | |
| A | CHEMICAL ABSTRACTS, vol. 71, no. 25, 22. Dezember 1969, Columbus, Ohio, US; abstract no. 123637M, Seite 373 ; <br> * Zusammenfassung * <br> & JP-A-44 013 692 (K. KONO) * <br> – – – – – | 1-7 | |

|  |
|---|
| **RECHERCHIERTE SACHGEBIETE (Int. Cl.5)** |
| C 07 C |

Der vorliegende Recherchenbericht wurde für alle Patentansprüche erstellt

| Recherchenort | Abschlußdatum der Recherche | Prüfer |
|---|---|---|
| Den Haag | 16 Januar 92 | SANCHEZ Y GARCIA J.M |